# EUROPEAN PATENT SPECIFICATION

(11) **EP 1 877 367 B1**
(45) Date of publication and mention of the grant of the patent: **14.10.2009**
(21) Application number: 06724540.7
(22) Date of filing: 24.04.2006
(51) Int. Cl.: C07C 271/24, C07D 307/68, C07D 307/85, C07D 213/81, C07D 213/82, C07D 231/38, C07D 307/32, C07D 295/08, C07D 211/58, C07C 309/14, C07D 233/54, A61P 1/00, A61P 13/00, A61P 25/00, A61K 31/325

(54) **ACETYLENE DERIVATIVES**
ACETYLENDERIVATE
DERIVES ACETYLENIQUES

(30) Priority: 25.04.2005 GB 0508314
(43) Date of publication of application: 16.01.2008
(73) Proprietor: Novartis AG, 4056 Basel (CH); NOVARTIS-PHARMA GMBH, 1230 Vienna (AT)
(72) Inventor: GLATTHAR, Ralf, 79713 Bad Säckingen (DE); TROXLER, Thomas, J., CH-4246 Wahlen b. Laufen (CH)
(74) Representative: Vögeli-Lange, Regina
(86) International application number: PCT/EP2006/003768
(87) International publication number: WO 2006/114264

(56) References cited:
- WO-A-03/047581
- HUEBNER HARALD ET AL: "Conjugated enynes as nonaromatic catechol bioisosteres: Synthesis, binding experiments, and computational studies of novel dopamine receptor agonists recognizing preferentially the D3 subtype" JOURNAL OF MEDICINAL CHEMISTRY, vol. 43, no. 4, 24 February 2000 (2000-02-24), pages 756-762, XP002387283 ISSN: 0022-2623
- CHUA ET AL: "Cyclohexenyl- and dehydropiperidinyl-alkynyl pyridines as potent metabotropic glutamate subtype 5 (mGlu5) receptor antagonists" BIOORGANIC & MEDICINAL CHEMISTRY LETTERS, OXFORD, GB, vol. 15, no. 20, 15 October 2005 (2005-10-15), pages 4589-4593, XP005064658 ISSN: 0960-894X

## Description

The present invention relates to novel acetylene derivatives, their preparation, their use as pharmaceuticals and pharmaceutical compositions containing them.

More particularly the invention provides a compound of formula (I) 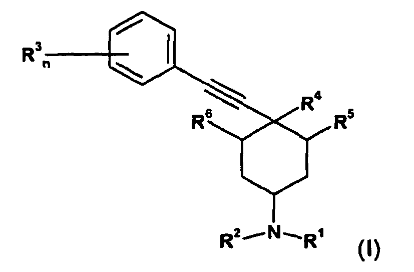 wherein
R¹ represents hydrogen and
R² represents an unsubstituted or substituted heterocycle having 3-11 ring atoms and 1-4 hetero atoms; the hetero atoms being selected from the group consisting of N, O, S, the substituents being selected from the group consisting of Oxo (=O), Hydroxy, Halogen, Amino, Nitro, Cyano, C₁₋₄ Alkyl, C₁₋₄ Alkoxy, C₁₋₄ Alkoxyalkyl, C₁₋₄ Alkoxycarbonyl, C₁₋₄ Alkoxycarbonylalkyl, C₁₋₄ Halogenalkyl, C₆₋₁₀ Aryl, Halogen- C₆₋₁₀ Aryl, C₆₋₁₀ Aryloxy, C₆₋₁₀-Aryl-C₁₋₄ alkyl, or
R¹ represents hydrogen and
R² represents phenyl or substituted phenyl, the substituents being selected from the group consisting of Hydroxy, Amino, Halogen, Nitro, Cyano, C₁₋₄ Alkyl, C₁₋₄ Alkoxy, C₁₋₄ Alkoxyalkyl, C₁₋₄ Alkoxycarbonyl, C₁₋₄ Alkoxycarbonylalkyl, C₁₋₄ Halogenalkyl, C₆₋₁₀ Aryl, Halogen- C₆₋₁₀ Aryl, C₆₋₁₀ Aryloxy, C₆₋₁₀-Aryl-C₁₋₄ alkyl; or
R¹ represents hydrogen and
R² represents C(O)R²¹ wherein
R²¹ represents unsubstituted or substituted C₁₋₄ alkyl, the substituents being selected from the group consisting of halogen, nitro, amino, hydroxy, C₆₋₁₀ Aryl, Halogen-C₆₋₁₀ Aryl, C₁₋₄Alkyl -C₆₋₁₀ Aryl, C₁₋₄Alkoxy -C₆₋₁₀ Aryl, C₁₋₄Halogenalkyl -C₆₋₁₀ Aryl; or
R²¹ represents unsubstituted or substituted C₁₋₄ alkoxy, the substituents being selected from the group consisting of halogen, nitro, amino, hydroxy, C₆₋₁₀ Aryl, Halogen-C₆₋₁₀ Aryl, C₁₋₄Alkyl -C₆₋₁₀ Aryl, C₁₋₄Alkoxy -C₆₋₁₀ Aryl, C₁₋₄Halogenalkyl -C₆₋₁₀ Aryl; or
R²¹ represents unsubstituted or substituted heterocycle having 3-11 ring atoms and 1-4 hetero atoms, the hetero atoms being selected from the group consisting of N, O, S, the substituents being selected from the group consisting of Oxo (=O), Hydroxy, Halogen, Amino, Nitro, Cyano, Cyano, C₁₋₄ Alkyl, C₁₋₄ Alkoxy, C₁₋₄ Alkoxyalkyl, C₁₋₄ Alkoxycarbonyl, C₁₋₄ Alkoxycarbonylalkyl, C₁₋₄ Halogenalkyl, C₆₋₁₀ Aryl, Halogen- C₆₋₁₀ Aryl, C₆₋₁₀ Aryloxy, C₆₋₁₀-Aryl-C₁₋₄ alkyl; or
R²¹ represents unsubstituted or substituted phenyl, the substituents being selected from the group consisting of Hydroxy, Amino, Halogen, Nitro, Cyano, C₁₋₄ Alkyl, C₁₋₄ Alkoxy, C₁₋₄ Alkoxyalkyl, C₁₋₄ Alkoxycarbonyl, C₁₋₄ Alkoxycarbonylalkyl, C₁₋₄ Halogenalkyl, C₆₋₁₀ Aryl, Halogen- C₆₋₁₀ Aryl, C₆₋₁₀ Aryloxy, C₆₋₁₀-Aryl-C₁₋₄ alkyl; or
R¹ and R² together with the nitrogen atom form unsubstituted or substituted heterocycle having 3-11 ring atoms and 0-3 additional hetero atoms; the hetero atoms being selected from the group consisting of N, O, S; the substituents being selected from the group consisting of Oxo (=O), Hydroxy, Halogen, Amino, Nitro, Cyano, C₁₋₄ Alkyl, C₁₋₄ Alkoxy, C₁₋₄ Alkoxyalkyl, C₁₋₄ Alkoxycarbonyl, C₁-₄ Alkoxycarbonylalkyl, C₁₋₄ Halogenalkyl, C₆₋₁₀ Aryl, Halogen- C₆₋₁₀ Aryl, C₆₋₁₀ Aryloxy, C₆₋₁₀ -Aryl-C₁₋₄ alkyl;
R³ represents (C₁₋₄)alkyl, or halagen,
n represents 1,
R⁴ represents OH and
R⁵ and R⁶ represent H
in free base or acid addition salt form.

WO 03/047581 describes acetylene derivatives having mGluR5 antagonistic activity.

In the present specification, the following definitions shall apply if no specific other definition is given:
"Alkyl" represents a straight-chain or branched-chain alkyl group, for example, methyl, ethyl, n- or iso-propyl, n-, iso-, sec- or tert-butyl, with particular preference given to methyl, ethyl, n-propyl and iso-propyl.
"Alkandiyl" represents a straight-chain or branched-chain alkandiyl group bound by two different Carbon atoms to the molecule, it preferably represents a straight-chain or branched-chain C₁₋₆alkandiyl; for example, methandiyl (-CH₂-), 1 ,2-ethanediyl (-CH₂-CH₂-), 1,1-ethanediyl ((-CH(CH₃)-), 1,1-, 1,2-, 1,3-propanediyl and 1,1-, 1,2-, 1,3-, 1,4-butanediyl, with particular preference given to methandiyl, 1,1-ethanediyl, 1,2-ethanediyl, 1,3-propanediyl, 1,4-butanediyl.
Each alkyl part of "alkoxy". "alkoxyalkyl", "alkoxycarbonyl", "alkoxycarbonylalkyl" and "halogenalkyl" shall have the same meaning as described in the above-mentioned definition of "alkyl".
"Alkenyl" represents a straight-chain or branched-chain alkenyl group, preferably C₂₋₆ alkenyl, for example, vinyl, allyl, 1-propenyl, isopropenyl, 2-butenyl, 2-pentenyl, 2-hexenyl, etc. and preferably represents C₂₋₄ alkenyl.
"Alkendiyl" represents a straight-chain or branched-chain alkendiyl group bound by two different Carbon atoms to the molecule, it preferably represents a straight-chain or branched-chain C₂₋₆ alkandiyl; for example, -CH=CH-, -CH=C(CH₃)-, -CH=CH-CH₂-,-C(CH₃)=CH-CH₂-, -CH=C(CH₃)-CH₂-, -CH=CH-C(CH₃)H-, -CH=CH-CH=CH-, -C(CH₃)=CH-CH=CH-, -CH=C(CH₃)-CH=CH-, with particular preference given to -CH=CH-CH₂-, -CH=CH-CH=CH-.
"Alkynyl" represents a straight-chain or branched-chain alkynyl group, preferably C₂₋₆alkynyl, for example, ethenyl, propargyl, 1-propynyl, isopropenyl, 1- (2- or 3) butynyl, 1- (2- or 3) pentenyl, 1- (2- or 3) hexenyl, etc. ,preferably represents C₂₋₄alkynyl and particularly preferably represents ethynyl.
"Aryl" represents an aromatic hydrocarbon group, preferably a C₆₋₁₀ aromatic hydrocarbon group; for example phenyl, naphthyl, especially phenyl.
"Aralkyl" denotes an "Aryl" bound to an "Alkyl" (both as defined above) an represents, for example benzyl, α-methylbenzyl, 2-phenylethyl, α,α-dimethylbenzyl, especially benzyl.
"Heterocycle" represents a saturated, partly saturated or aromatic ring system containing at least one hetero atom. Preferably, heterocycles consist of 3 to 11 ring atoms of which 1-3 ring atoms are hetero atoms. Heterocycles may be present as a single ring system or as bicyclic or tricyclic ring systems; preferably as single ring system or as benz-annelated ring system. Bicyclic or tricyclic ring systems may be formed by annelation of two or more rings, by a bridging atom, e.g. Oxygen, sulfur, nitrogen or by a bridging group, e.g. alkandediyl or alkenediyl. A Heterocycle may be substituted by one or more substituents selected from the group consisting of Oxo (=O), Halogen, Nitro, Cyano, Alkyl, Alkandiyl, Alkenediyl, Alkoxy, Alkoxyalkyl, Alkoxycarbonyl, Alkoxycarbonylalkyl, Halogenalkyl, Aryl, Aryloxy, Arylalkyl. Examples of heterocyclic moieties are: pyrrole, pyrroline, pyrrolidine, pyrazole, pyrazoline, pyrazolidine, imidazole, imidazoline, imidazolidine, triazole, triazoline, triazolidine, tetrazole, furane, dihydrofurane, tetrahydrofurane, furazane (oxadiazole), dioxolane, thiophene, dihydrothiophene, tetrahydrothiophene, oxazole, oxazoline, oxazolidine, isoxazole, isoxazoline, isoxazolidine, thiazole, thiazoline, thiazlolidine, isothiazole, istothiazoline, isothiazolidine, thiadiazole, thiadiazoline, thiadiazolidine, pyridine, piperidine, pyridazine, pyrazine, piperazine, triazine, pyrane, tetrahydropyrane, thiopyrane, tetrahydrothiopyrane, oxazine, thiazine, dioxine, morpholine, purine, pterine, and the corresponding benz-annelated heterocycles, e.g. indole, isoindole, cumarine, cumaronecinoline, isochinoline, cinnoline and the like.
"Hetero atoms" are atoms other than Carbon and Hydrogen, preferably Nitrogen (N), Oxygen (O) or Sulfur (S).
"Halogen" represents Fluoro, Chloro, Bromo or lodo, preferably represents Fluoro, Chloro or Bromo and particularly preferably represents Chloro.

Compounds of formula (I) exist in free or acid addition salt form. In this specification, unless otherwise indicated, language such as "compounds of formula (I)" is to be understood as embracing the compounds in any form, for example free base or acid addition salt form. Salts which are unsuitable for pharmaceutical uses but which can be employed, for example, for the isolation or purification of free compounds of formula (I) , such as picrates or perchlorates, are also included. For therapeutic use, only pharmaceutically acceptable salts or free compounds are employed (where applicable in the form of pharmaceutical preparations), and are therefore preferred.

On account of the asymmetrical carbon atom(s) that may be present in the compounds of formula (I) and their salts, the compounds may exist in optically active form or in form of mixtures of optical isomers, e.g. in form of racemic mixtures or diastereomeric mixtures. All optical isomers and their mixtures, including the racemic mixtures, are part of the present invention. Preferred compounds of formula (I) have trans configuration in respect to R⁴ and N.

Preferred substituents, preferred ranges of numerical values or preferred ranges of the radicals present in the formula (I) and the corresponding intermediate compounds are defined below.
- R³: particularly preferably represents Fluoro, Chloro, methyl.
- R²: particularly preferably represents an unsubstituted, a single or twofold substituted heterocycle having 5 - 9 ring atoms and 1 - 3 hetero atoms; the hetero atoms being selected from the group consisting of N, O; the substituents being selected from the group consisting of Halogen, C₁₋₄ Alkyl, C₁₋₄ Alkoxy, C₆₋₁₀ Aryl, Halogen-C₆₋₁₀ Aryl, C₆₋₁₀ Aryloxy, C₆₋₁₀-Aryl-C₁₋₄ alkyl.
- R²: particularly preferably represents an unsubstituted, a single or twofold substituted phenyl, the substituents being selected from the group consisting of Halogen, Cyano, C₁₋₄ Alkyl, C₁₋₄ Alkoxy, phenyl, halogenphenyl, phenyloxy, benzyl, pheylethyl.
- R²¹: particularly preferably represents C₁₋₄ alkyl or substituted C₁₋₄ alkyl, the substituents being selected from the group consisting of Halogen, C₁₋₄ Alkyl, C₁₋₄ Alkoxy, C₆₋₁₀ Aryl, Halogen-C₆₋₁₀ Aryl, C₆₋₁₀ Aryloxy, C₆₋₁₀-Aryl-C₁₋₄ alkyl.
- R²¹: particularly preferably represents C₁-C₁₋₄ alkoxy or substituted C₁₄ alkoxy, the substituents being selected from the group consisting of Halogen, C₁₋₄ Alkyl, C₁₋₄ Alkoxy, C₆₋₁₀ Aryl, Halogen-C₆₋₁₀ Aryl, C₆₋₁₀ Aryloxy, C₆₋₁₀-Aryl-C₁₋₄ alkyl.
- R²¹: particularly preferably represents a single or twofold substituted heterocycle having 5-9 ring atoms and 1 - 3 hetero atoms, the hetero atoms being selected from the group consisting of N, O; the substituents being selected from the group consisting of Halogen, C₁₋₄ Alkyl, C₁₋₄ Alkoxy, C₆₋₁₀ Aryl, Halogen-C₆₋₁₀ Aryl, C₆₋₁₀ Aryloxy, C₆₋₁₀-Aryl-C₁₋₄ alkyl.
- R²¹: particularly preferably represents an unsubstituted, a single or twofold substituted phenyl, the substituents being selected from the group consisting of Halogen, Cyano, C₁₋₄ Alkyl, C₁₋₄ Alkoxy, phenyl, halogenphenyl, phenyloxy, benzyl, pheylethyl.
- R¹ and R²: together with the nitrogen atom further particularly preferably form a single or twofold substituted heterocycle having 5 - 9 ring atoms and 0 - 2 additional hetero atoms; the hetero atoms being selected from the group consisting of N, O; the substituents being selected from the group consisting of Halogen, C₁₋₄ Alkyl, C₁₋₄ Alkoxy, C₆₋₁₀ Aryl, Halogen-C₆₋₁₀ Aryl, C₆₋₁₀ Aryloxy, C₆₋₁₀-Aryl-C₁₋₄ alkyl.
- R²: very particularly preferably represents chlorphenyl or dichlorphenyl.
- R²¹: very particularly preferably represents methoxy, tert.butyloxy.
- R²¹: very particularly preferably represents furyl, benzfuranyl, pyridyl.

The abovementioned general or preferred radical definitions apply both to the end products of the formula (I) and also, correspondingly, to the starting materials or intermediates required in each case for the preparation. These radical definitions can be combined with one another at will, i.e. including combinations between the given preferred ranges. Further, individual definitions may not apply.

Preference according to the invention is given to compounds of the formula (I) which contain a combination of the meanings mentioned above as being preferred.

Particular preference according to the invention is given to compounds of the formula (I) which contain a combination of the meanings listed above as being particularly preferred.

Very particular preference according to the invention is given to the compounds of the formula (I) which contain a combination of the meanings listed above as being very particularly preferred.

A further preferred group of compounds of formula (I) are compounds wherein R³ is in the meta-position.

In a further aspect, the invention provides a process for the production of the compounds of formula I and their salts, whichⁱcomprises the step of
a) for the production of a compound of formula (I) wherein R⁴ is hydroxy, R⁵ and R⁶ are hydrogen, reacting a compound of formula (II) 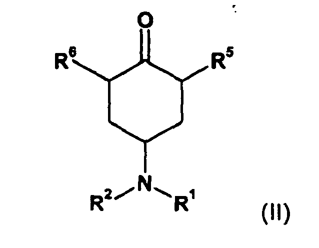 wherein R¹, R², R³, R⁴ are as defined above, with a compound of formula (III) 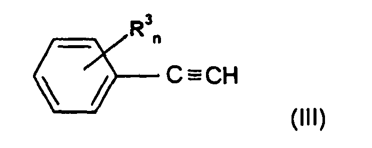 wherein R³ and n are as defined above, or
b) for the production of a compound of formula (I) wherein i) R⁴ represents hydroxy, R¹ represents hydrogen and R² represents an unsubstituted or substituted heterocycle or ii) R¹ represents hydrogen and R² represents aryl or substituted aryl, by reductive amination of a compound of formula (IV) 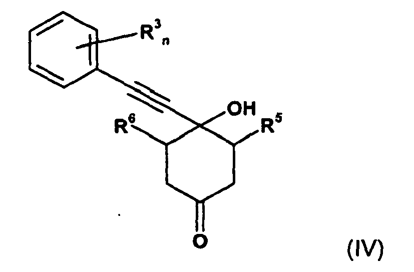 wherein R⁶, R⁵, R³, n are as defined above, with a compound of formula (V) 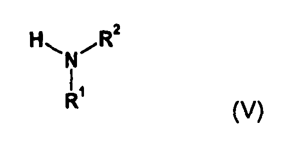 wherein R¹ and R² are as defined above, or
c) for the production of a compound of formula (I) wherein R⁴ represents hydroxy, R¹ and R² together with the nitrogen atom form an unsubstituted or substituted heterocycle, by cyclocondensation of a compound of formula (VI) 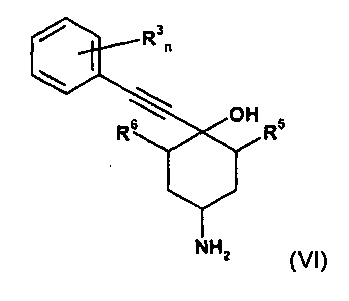
and recovering the resulting compound of formula (I) in free base or acid addition salt form.

The reaction of process a) and b) and c) can be effected according to conventional methods, e.g. as described in the Examples.

The reaction of process b) is performed in the presence of a reducing agent, such as a alkalialkyle, methalhydride or a borohydride, preferably a borohydride such as sodiumtriacetoxyborohydride.

A so obtained compound of formula (I) can be converted into another compound of formula (I) according to conventional methods.

Generally, the starting materials for manufacturing compounds of formula (I) are known or obtainable according to known processes.

Compounds of formula (IV) are obtainable by reacting a compound of formula 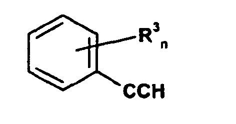 wherein R³, n are as defined above with a formula 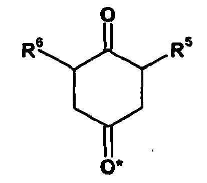 wherein R⁶, R⁵, are as defined above and O* represents the oxygen of a carbonyl-group that is protected, e.g. by acetale-formation.

The following considerations apply to the individual reaction steps described above:
a) One or more functional groups, for example carboxy, hydroxy, amino, or mercapto, may need to be protected in the starting materials by protecting groups. The protecting groups employed may already be present in precursors and should protect the functional groups concerned against unwanted secondary reactions, such as acylations, etherifications, esterifications, oxidations, solvolysis, and similar reactions. It is a characteristic of protecting groups that they lend themselves readily, i.e. without undesired secondary reactions, to removal, typically by solvolysis, reduction, photolysis or also by enzyme activity, for example under conditions analogous to physiological conditions, and that they are not present in the end-products. The specialist knows, or can easily establish, which protecting groups are suitable with the reactions mentioned hereinabove and hereinafter. The protection of such functional groups by such protecting groups, the protecting groups themselves, and their removal reactions are described for example in standard reference works, such as J. F. W. McOmie, "Protective Groups in Organic Chemistry", Plenum Press, London and New York 1973, in T. W. Greene, "Protective Groups in Organic Synthesis", Wiley, New York 1981, in "The Peptides"; Volume 3 (editors: E. Gross and J. Meienhofer), Academic Press, London and New York 1981, in "Methoden der organischen Chemie" (Methods of organic chemistry), Houben Weyl, 4th edition, Volume 15/I, Georg Thieme Verlag, Stuttgart 1974, in H.-D. Jakubke and H. Jescheit, "AminosAuren, Peptide, Proteine" (Amino acids, peptides, proteins), Verlag Chemie, Weinheim, Deerfield Beach, and Basel 1982, and in Jochen Lehmann, "Chemie der Kohlenhydrate: Monosaccharide und Derivate" (Chemistry of carbohydrates: monosaccharides and derivatives), Georg Thieme Verlag, Stuttgart 1974.
b) Acid addition salts may be produced from the free bases in known manner, and vice-versa. Compounds of formula (I) in optically pure form can be obtained from the corresponding racemates according to well-known procedures, e.g. HPLC with chiral matrix. Alternatively, optically pure starting materials can be used.
c) Stereoisomeric mixtures, e.g. mixtures of diastereomers, can be separated into their corresponding isomers in a manner known per se by means of suitable separation methods. Diastereomeric mixtures for example may be separated into their individual diastereomers by means of fractionated crystallization, chromatography, solvent distribution, and similar procedures. This separation may take place either at the level of a starting compound or in a compound of formula I itself. Enantiomers may be separated through the formation of diastereomeric salts, for example by salt formation with an enantiomer-pure chiral acid, or by means of chromatography, for example by HPLC, using chromatographic substrates with chiral ligands.
d) Suitable diluents for carrying out the above- described are especially inert organic solvents. These include, in particular, aliphatic, alicyclic or aromatic, optionally halogenated hydrocarbons, such as, for example, benzine, benzene, toluene, xylene, chlorobenzene, dichlorobenzene, petroleum ether, hexane, cyclohexane, dichloromethane, chloroform, carbon tetrachloride; ethers, such as diethyl ether, diisopropyl ether, dioxane, tetrahydrofuran or ethylene glycol dimethyl ether or ethylene glycol diethyl ether; ketones, such as acetone, butanone or methyl isobutyl ketone; nitriles, such as acetonitrile propionitrile or butyronitrile; amides, such as N,N-dimethylformamide, N,N-dimethylacetamide, N-methyl-formanilide, N-methyl-pyrrolidone or hexamethylphosphoric triamide; esters, such as methyl acetate or ethyl acetate, sulphoxides, such as dimethyl sulphoxide, alcohols, such as methanol, ethanol, n- or i-propanol, ethylene glycol monomethyl ether, ethylene glycol monoethyl ether, diethyelene glycol monomethyl ether, diethylene glycol monoethyl ether. Further, mixtures of diluents may be employed. Depending on the starting materials, reaction conditions and auxiliaries, water or diluents constaining water may be suitable. It is also possible to use one a starting material as diluent simultaneously.
e) Reaction temperatures can be varied within a relatively wide range. In general, the processes are carried out at temperatures between 0°C and 150°C, preferably between 10°C and 120°C. Deprotonation reactions can be varied within a relatively wide range. In general, the processes are carried out at temperatures between -150°C and +50°C, preferably between -75°C and 0°C.
f) The reactions are generally carried out under atmospheric pressure. However, it is also possible to carry out the processes according to the invention under elevated or reduced pressure - in general between 0.1 bar and 10 bar.
g) Starting materials are generally employed in approximately equimolar amounts. However, it is also possible to use a relatively large excess of one of the components. The reaction is generally carried out in a suitable diluent in the presence of a reaction auxiliary, and the reaction mixture is generally stirred at the required temperature for a number of hours.
h) Work-up is carried out by customary methods (cf. the Preparation Examples).

Compounds of formula (I) and their pharmaceutically acceptable acid addition salts, hereinafter referred to as agents of the invention, exhibit valuable pharmacological properties and are therefore useful as pharmaceuticals.

In particular, the agents of the invention exhibit a marked and selective modulating, especially antagonistic, action at human metabotropic glutamate receptors (mGluRs). This can be determined in vitro for example at recombinant human metabotropic glutamate receptors, especially PLC-coupled subtypes thereof such as mGluR5, using different procedures like, for example, measurement of the inhibition of the agonist induced elevation of intracellular Ca²⁺ concentration in accordance with L. P. Daggett et al., Neuropharm. Vol. 34, pages 871-886 (1995), P. J. Flor et al., J. Neurochem. Vol. 67, pages 58-63 (1996) or by determination to what extent the agonist induced elevation of the inositol phosphate turnover is inhibited as described by T. Knoepfel et al., Eur. J. Pharmacol. Vol. 288, pages 389-392 (1994), L. P. Daggett et al., Neuropharm. Vol. 67, pages 58-63 (1996) and references cited therein. Isolation and expression of human mGluR subtypes are described in US-Patent No. 5,521,297. Selected agents of the invention show IC50 values for the inhibition of the agonist (e.g. glutamate or quisqualate) induced elevation of intracellular Ca2+ concentration or the agonist (e.g. glutamate or quisqualate) induced inositol phosphate turnover, measured in recombinant cells expressing hmGluR5a of about 1 nM to about 50 µM.

The agents of the invention are therefore useful in the treatment of disorders associated with irregularities of the glutamatergic signal transmission, of the gastro-intestinal and urinary tract and of nervous system disorders mediated full or in part by mGluR5.

Disorders associated with irregularities of the glutamatergic signal transmission are for example epilepsy, cerebral ischemias, especially acute ischemias, ischemic diseases of the eye, muscle spasms such as local or general spasticity, skin disorders, obesity disorders and, in particular, convulsions or pain.

Disorders of the gastro-intestinal tract include post-operative ileus, functional gastro-intestinal disorders (FGID) as for example functional dyspepsia (FD), gastro-esophageal reflux disease (GERD), irritable bowel syndrome (IBS), functional bloating, functional diarrhea, chronic constipation, functional disturbancies of the biliary tract as well as other conditions according to Gut 1999; Vol. 45 Suppl. II.

Disorders of the Urinary Tract comprise conditions associated with pain and/or discomfort of the urinary tract and overactive bladder (OAB).

Nervous system disorders mediated full or in part by mGluR5 are for example acute, traumatic and chronic degenerative processes of the nervous system, such as Parkinson's disease, senile dementia, Alzheimer's disease, Huntington's chorea, amyotrophic lateral sclerosis, multiple sclerosis and fragile X syndrome, psychiatric diseases such as schizophrenia and anxiety, depression, pain, itch and drug abuse. Anxiety related disorders includes panic disorders, social anxiety, obsessive compulsive disorders (OCD), post traumatic stress disorders (ATSD), generalized anxiety disorders (GAD), phobias.

The usefulness of the agents of the invention in the treatment of the above-mentioned disorders can be confirmed in a range of standard tests including those indicated below: Activity of the agents of the invention in anxiety can be demonstrated in standard models such as the stress-induced hyperthermia in mice [cf. A. Lecci et al., Psychopharmacol. 101, 255-261]. At doses of about 0.1 to about 30 mg/kg p.o., selected agents of the invention reverse the stress-induced hyperthermia.

At doses of about 4 to about 50 mg/kg p.o., selected agents of the invention show reversal of Freund complete adjuvant (FCA) induced hyperalgesia [cf. J. Donnerer et al., Neuroscience 49, 693-698 (1992) and C.J. Woolf, Neuroscience 62, 327-331 (1994)].

For all the above mentioned indications, the appropriate dosage will of course vary depending upon, for example, the compound employed, the host, the mode of administration and the nature and severity of the condition being treated. However, in general, satisfactory results in animals are indicated to be obtained at a daily dosage of from about 0.5 to about 100 mg/kg animal body weight. In larger mammals, for example humans, an indicated daily dosage is in the range from-about 5 to 1500 mg, preferably about 10 to about 1000 mg of the compound conveniently administered in divided doses up to 4 times a day or in sustained release form.

In accordance with the foregoing, the present invention also provides an agent of the invention for use as a pharmaceutical, e.g. in the treatment of disorders associated with irregularities of the glutamatergic signal transmission, and of nervous system disorders mediated full or in part by mGluR5.

The invention also provides the use of an agent of the invention, in the treatment of disorders associated with irregularities of the glutamatergic signal transmission, and of nervous system disorders mediated full or in part by mGluR5.

Furthermore the invention provides the use of an agent of the invention for the manufacture of a pharmaceutical composition designed for the treatment of disorders associated with irregularities of the glutamatergic signal transmission, and of nervous system disorders mediated full or in part by mGluR5.

In a further aspect the invention relates to a method of treating disorders mediated full or in part by mGluR5, which method comprises administering to a warm-blooded organism in need of such treatment a therapeutically effective amount of an agent of the invention.

Moreover the invention relates to a pharmaceutical composition comprising an agent of the invention in association with one or more pharmaceutical carrier or one or more pharmaceutically acceptable diluent.

The pharmaceutical compositions according to the invention are compositions for enteral, such as nasal, rectal or oral, or parenteral, such as intramuscular or intravenous, administration to warm-blooded animals (human beings and animals) that comprise an effective dose of the pharmacological active ingredient alone or together with a significant amount of a pharmaceutically acceptable carrier. The dose of the active ingredient depends on the species of warm-blooded animal, body weight, age and individual condition, individual pharmacokinetic data, the disease to be treated and the mode of administration.

The pharmaceutical compositions comprise from approximately 1 % to approximately 95%, preferably from approximately 20% to approximately 90%, active ingredient. Pharmaceutical compositions according to the invention may be, for example, in unit dose form, such as in the form of ampoules, vials, suppositories, dragées, tablets or capsules.

The pharmaceutical compositions of the present invention are prepared in a manner known per se, for example by means of conventional dissolving, lyophilizing, mixing, granulating or confectioning processes.

The preferred agents of the invention include the 4-(3-Chloro-phenylamino)-1-(3-chlor o-phenylethynyl)-cyclohexanol free base or pharmaceutically acceptable acid addition salt form.

4-(3-Chloro-phenylamino)-1-(3-chlor o-phenylethynyl)-cyclohexanol inhibits the quisqualate-induced inositol phosphate turnover in hmGluR5 expressing cells with an IC₅₀ concentration of 4000 nM.

Further, properly isotope-labeled agents of the invention exhibit valuable properties as histopathological labeling agents, imaging agents and/or biomarkers, hereinafter "markers", for the selective labeling of the metabotropic glutamate receptor subtype 5 (mGlu5 receptor). More particularly the agents of the invention are useful as markers for labeling the central and peripheral mGlu5 receptors *in vitro* or *in vivo.* In particular, compounds of the invention which are properly isotopically labeled are useful as PET markers. Such PET markers are labeled with one or more atoms selected from the group consisting of ¹¹C, ¹³N, ¹⁵O, ¹⁸F.

The agents of the invention are therefore useful, for instance, for determining the levels of receptor occupancy of a drug acting at the mGlu5 receptor, or diagnostic purposes for diseases resulting from an imbalance or dysfunction of mGlu5 receptors, and for monitoring the effectiveness of pharmacotherapies of such diseases.

In accordance with the above, the present invention provides an agent of the invention for use as a marker for neuroimaging.

In a further aspect, the present invention provides a composition for labeling brain and peripheral nervous system structures involving mGlu5 receptors *in vivo* and *in vitro* comprising an agent of the invention.

In still a further aspect, the present invention provides a method for labeling brain and peripheral nervous system structures involving mGlu5 receptors *in vitro* or *in vivo,* which comprises contacting brain tissue with an agent of the invention.

The method of the invention may comprise a further step aimed at determining whether the agent of the invention labeled the target structure. Said further step may be effected by observing the target structure using positron emission tomography (PET) or single photon emission computed tomography (SPECT), or any device allowing detection of radioactive radiations.

The following non-limiting Examples illustrate the invention. A list of Abbreviations used is given below.

| | |
|---|---|
| BOC | tert-butoxycarbonyl |
| n-BuLi | *n*-butyllithium |
| DCM | dichloromethane |
| DMF | N,N'-dimethylformamide |
| EDC | 1-ethyl-3-[3-(dimethylamino)propyl)-carbodiimide hydrochloride |
| EtOAc | ethylacetate |
| h | hours |
| HCl | hydrochloric acid |
| HOBt | hydroxybenzotriazole |
| HPLC | high pressure liquid chromatography |
| min | minutes |
| Mp | melting point |
| MS | mass spectroscopy |
| MTBE | methyl-*tert*.-butylether |
| Rf | retention factor (Thin Layer Chromatography) |
| rt | room temperature |
| Rt | retention time |
| TFA | trifluoroacetic acid |
| THF | tetrahydrofuran |

### Example 1: trans-[4-(3-Chloro-phenylethynyl)-4-hydroxy-cyclohexyl]-carbamic acid methyl ester and cis-[4-(3-Chloro-phenylethynyl)-4-hydroxy-cyclohexyl]-carbamic acid methyl ester

To a solution of *n-*buthyl lithium (5.5 ml of 1.6 M solution in hexane, 8.76 mmol, 1.0 eq) in THF (10 ml) at - 70 °C under argon is added a solution of 1-Chloro-3-ethynyl-benzene (1.22 g, 8.76 mmol, 1.0 eq) in THF (7 ml). After stirring the reaction mixture for 30 minutes at - 70 °C a solution of (4-oxo-cyclohexyl)-carbamic acid methyl ester (1.50 g, 8.76 mmol, 1 eq) in THF (7 ml) is added and the mixture is stirred for another 30 min. The solution is diluted with 10 % aqueous ammonium chloride solution (3 ml) and EtOAc (5 ml). The organic layer is washed with 1 N aqueous HCl solution (3 x 5 ml) dried over sodium sulfate and the solvent is evaporated. The obtained mixture of *cis*/*trans*-isomers could be separated on silica (Flashmaster, EtOAc/hexane) to afford single isomers in a 1:1 ratio (0.45 g, 17 %).
*trans*-isomer:
   MS (LC/MS): 330 [M+H]
   TLC Rf: 0.42 (EtOAc/hexane = 1/1)
*cis*-isomer:
   MS (LC/MS): 330 [M+Na]
   TLC Rf: 0.45 (EtOAc/hexane = 1/1)

Following the same procedure, the following compounds are obtained:

### Example 1.1: trans-[4-(4-Chloro-phenylethynyl)-4-hydroxy-cyclohexyl]-carbamic acid methyl ester and cis-[4-(4-Chloro-phenylethynyl)-4-hydroxy-cyclohexyl]-carbamic acid methyl ester

*trans*-isomer:
   MS (LC/MS): 330 [M+Na]
   TLC Rf: 0.37 (EtOAc/hexane = 1/1)
*cis*-isomer:
   MS (LC/MS): 330 [M+Na]
   TLC Rf: 0.43 (EtOAc/hexane = 1/1)

### Example 1.2: cis-[4-(3-Chloro-phenylethynyl)-4-hydroxy-cyclohexyl]-carbamic acid tert-butyl ester and trans-[4-(3-Chloro-phenylethynyl)-4-hydroxy-cyclohexyl]-arbamic acid tert-butyl ester

To a solution of *n*-buthyl lithium (3.7 ml of 1.6 M solution in hexane, 5.90 mmol, 1.01 eq) in THF (60 ml) at - 70 °C under argon is added a solution of 1-Chloro-3-ethynyl-benzene (0.83 g, 6.05 mmol, 1.04 eq) in THF (20 ml). After stirring the reaction mixture for 30 minutes at-70 °C a solution of (4-oxo-cyclohexyl)-carbamic acid tert-butyl ester (1.24 g, 5.81 mmol, 1 eq) in THF (20 ml) is added and the mixture is stirred for another 10 h. The solution is diluted with 10 % aqueous ammonium chloride solution (50 ml) and EtOAc (100 ml). The organic layer is washed with 1 N aqueous HCl solution (3 x 20 ml) dried over sodium sulfate and the solvent is evaporated. The obtained mixture of *cis*/*trans*-isomers could be separated on silica (Flashmaster, EtOAc/hexane) to afford single isomers in a 10:1 *cis*/*trans*-ratio (1.12 g, 55%).
*cis*-isomer:
   MS (LC/MS): 372 [M+Na]
   TLC Rf: 0.60 (EtOAc/hexane = 1/1)
*trans*-isomer:
   MS (LC/MS): 372 [M+Na]
   TLC Rf: 0.23 (EtOAc/hexane = 1/2)

### Example 1.3: cis-Furan-3-carboxylic acid [4-(3-chloro-phenylethynyl)-4-hydroxy-cyclohexyl]-amide

To a solution of *cis*-[4-(3-Chloro-phenylethynyl)-4-hydroxy-cyclohexyl]-carbamic acid tert-butyl ester (92 mg, 0.26 mmol) in DCM (2 ml) at 0°C is added a 4 N solution of HCl in dioxane (0.5 ml). After stirring the reaction mixture for 1 h at rt, the solvent is evaporated to afford the crude amine as it's hydrochlorid salt. This material was dissolved in DCM (3 ml) and Furane-3-carboxylic acid (35.0 mg, 0.31 mmol, 1.2 eq) is added, followed by EDC (61 mg, 0.31 mmol, 1.2 eq), HOBt (43 mg, 0.31 mmol, 1.2 eq) and triethylamine (0.11 ml, 1.30 mmol, 5 eq). After stirring at rt for 23 h, 1 N aqueous HCl (2 ml) is added and the solution is extracted with EtOAc (3 x 7 ml). Combined organic layers are washed with 10 % aqueous hydrogen carbonate solution (3 ml) dried over sodium sulfate and the solvent is evaporated. Resulting crude product is purified on silica (Flashmaster, EtOAc/hexane) to afford pure amide (23 mg, 25 %).
MS (LC/MS): 366 [M+Na]
TLC Rf: 0.40 (EtOAc/hexane = 1/1).

following procedure 1.3. the following compounds are obtained:

### Example 1.4: trans-Furan-3-carboxylic acid [4-(3-chloro-phenylethynyl)-4-hydroxycyclohexyl]-amide

MS (LC/MS): 344 [M+H]
TLC Rf: 0.19 (EtOAc/hexane = 1/1)

### Example 1.5: cis-Benzofuran-2-carboxylic acid [4-(3-chloro-phenylethynyl)-4-hydroxycyclohexyl]-amide

MS (LC/MS): 416 [M+Na]
TLC Rf: 0.55 (EtOAc/hexane = 1/1)

### Example 1.6: cis-Furan-2-carboxylic acid [4-(3-chloro-phenylethynyl)-4-hydroxy-cyclohexyl]-amide

MS (LC/MS): 366 [M+Na]
TLC Rf: 0.33 (EtOAc/hexane = 1/1)

### Example 1.7: cis-Pyridine-2-carboxylic acid [4-(3-chloro-phenylethynyl)-4-hydroxycyclohexyl]-amide

MS (LC/MS): 377 [M+Na]
TLC Rf: 0.32 (EtOAc/hexane = 1/1)

### Example 1.8: cis-N-[4-(3-Chloro-phenylethynyl)-4-hydroxy-cyclohexyl]-nicotinamide

MS (LC/MS): 355 [M+H]
TLC Rf: 0.06 (EtOAc/hexane = 1/1)

### Example 1.9: cis- N-[4-(3-Chloro-phenylethynyl)-4-hydroxy-cyclohexyl]-isonicotinamide

MS (LC/MS): 355 [M+H]
TLC Rf: 0.75 (EtOAc/hexane = 1/1)

Example 2.0: 1-(3-Chloro-phenylethynyl)-4-(5-methyl-1H-pyrazol-3-ylamino)-cyclohexanol A solution of 4-(3-Chloro-phenylethynyl)-4-hydroxy-cyclohexanone (70 mg, 0.281 mmol), 3-amino-5-methylpyrazole (27.3 mg, 0.281 mmol) and acetic acid (0.016 ml, 0.281 mmol) in 1,2-Dichloroethane (15 ml) is treated with sodiumtriacetoxy borohydride (83.5 mg, 0.394 mmol) and stirred for 21 h at room temperature. The mixture is diluted with EtOAc, washed with sodium bicarbonate and brine, dried (Na₂SO₄) and the solvent evaporated. Purification by chromatography on silica gel afforded a 1:1 cis/trans mixture of the title compound as an amorphous powder (26.2 mg, 28%).
MS (LC/MS): 330 [M+H].
TLC Rf: 0.08/0.16 (MeOH/DCM/Et₃N = 94/5/1)

The starting material was prepared as described hereafter:
i) 8-(3-Chloro-phenylethynyl)-1,4-dioxa-spiro[4.5]decan-8-ol
   1-Chloro-3-ethynyl-benzene (2.7 ml, 19.2 mmol) was dissolved in THF (250 ml) and cooled to -70°C. A solution of n-BuLi in hexanes (11.6 ml, 1.6 M, 19.0 mmol) was added within 0.5 h and the solution stirred for an additional hour at this temperature. A solution of 1,4-Dioxaspiro[4.5]decan-8-one (2.5 g, 18.3 mmol) in THF (30 ml) was added dropwise within 30' and the reaction mixture was stirred for another 5 h. After warming up t o rt, EtOAc was added and the mixture was washed with aqueous sodium bicarbonate and brine, dried and evaporated to afford an orange oil (8.43 g). Chromatography on silica gel afforded the title compound as a yellow oil (4.63 g, 86%).
ii) 4-(3-Chloro-phenylethynyl)-4-hydroxy-cyclohexanone
   A solution of 8-(3-Chloro-phenylethynyl)-1,4-dioxa-spiro[4.5]decan-8-ol (4.6 g, 15.7 mmol) and p-TsOH (598 mg) in acetone (50 ml) was stirred at 45 °C for 24 h. Dilution with EtOAc, washing with aqueous sodium bicarbonate and brine, drying and evaporation of the solvents afforded the crude product which was purified on silica gel afford the pure title compound (1.18 g, 30%).

Following the same procedure, the following compounds can be obtained:

### Example 2.1: 3-[4-(3-Chloro-phenylethynyl)-4-hydroxy-cyclohexylamino]-dihydro-furan-2-one

MS (LC/MS): 356 [M+Na]
TLC Rf: 0.45/0.55 (MeOH/DCM = 95/5)

### Example 2.2: 4-(3-Chloro-phenylamino)-1-(3-chlor o-phenylethynyl)-cyclohexanol

MS (ESI-MS): 360 [M]
TLC Rf: 0.58 (EtOAc/hexane = 1/1)

### Example 2.3: 1-(3-Chloro-phenylethynyl)-4-(3-methoxy-phenylamino)-cyclohexanol

MS (LC/MS): 356 [M+H]
TLC Rf: 0.36/0.48 (EtOAc/hexanes = 1/1)

### Example 2.4: 1-(3-Chloro-phenylethynyl)-4-(1H-pyrazol-3-ylamino)-cyclohexanol

MS (LC/MS): 316 [M+H]
TLC Rf: 0.67/0.75 (MeOH/DCM = 5/1)

### Example 2.5: 4-(4-Chloro-phenylamino)-1-(3-chlor o-phenylethynyl)-cyclohexanol

MS (LC/MS): 360 [M]
TLC Rf: 0.53 (EtOAc/hexane = 1/1)

### Example 2.6: 4-(3,5-Dichloro-phenylamino)-1-(3-chlor o-phenylethynyl)-cyclohexanol

MS (LC/MS): 394 [M+H]
Mp: 145-149 °C.

### Example 2.7: 1-(3-Chloro-phenylethynyl)-4-morpholin-4-yl-cyclohexanol

MS (LC/MS): 320 [M+H]
TLC Rf: 0.08/0.08 (MeOH/DCM = 95/5)

### Example 2.8: 1-(3-Chloro-phenylethynyl)-4-(1-methyl-piperidin-4-ylamino)-cyclohexanol

MS (LC/MS): 347 [M+H]
TLC Rf: 0.06/0.14 (MeOH/DCM/Et₃N = 94/5/1)

### Example 2.9: 4-(1-Aza-bicyclo[2.2.2]oct-3-ylamino)-l-(3-chloro-phenylethynyl)-cyclohexanol

MS (LC/MS): 359 [M+H]
TLC Rf: 0.07/0.14 (MeOH/DCM/Et₃N = 94/5/1)

### Example 2.10: 1-(3-Chloro-phenylethynyl)-4-(tetrahydro-pyran-4-ylamino)-cyclohexanol

MS (LC/MS): 334 [M+H]
TLC Rf: 0.50/0.50 (MeOH/DCM/Et₃N = 94/5/1)

### Example 2.11: trans-1-(3-Chloro-phenylethynyl)-4-imidazol-1-yl-cyclohexanol

A solution of *trans*-4-amino-1-(3-chloro-phenylethynyl)-cyclohexanol (75 mg, 0.3 mmol) in water (1.4 ml) was acidified with phorphoric acid to pH = 2.0. Dioxane (0.6 ml), paraformaldehyde (27 mg, 0.3 mmol) and glyoxal (40% aq. solution, 0.034 ml, 0.3 mmol) were added and the mixture heated to 80°. Ammonium chloride (19 mg, 0.3 mmol) was added and heating continued for 9 h. More paraformaldehyde (27 mg, 0.3 mmol), glyoxal (0.034 ml, 0.3 mmol) and ammonium chloride (19 mg, 0.3 mmol) was added and heating continued for 2 h. The mixture was cooled to room temperature and basified with 30% NaOH. Extraction with EtOAc, drying of the organic extracts with Na2SO4 and evaporation of the solvents afforded 75 mg of a crude product, which was purified by preparative TLC using EtOAc/EtOH/NH4OH 9:1:0.1 as mobile phase to afford pure *trans*-1-(3-chlorophenylethynyl)-4-imidazol-1-yl-cyclohexanol (15 mg, 17%).
MS (LC/MS): 301 [MH+], TLC Rf: 0.42 (EtOAc/EtOH/NH4OH 9:1:0.1).

## Claims

1. A compound of formula (I) 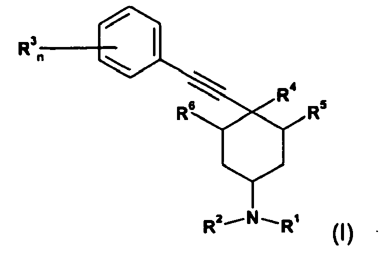 wherein
R¹ represents hydrogen and
R² represents an unsubstituted or substituted heterocycle having 3-11 ring atoms and 1 - 4 hetero atoms; the hetero atoms being selected from the group consisting of N, O, S, the substituents being selected from the group consisting of Oxo (=O), Hydroxy, Halogen, Amino, Nitro, Cyano, C₁₋₄ Alkyl, C₁₋₄ Alkoxy, C₁₋₄ Alkoxyalkyl, C₁₋₄ Alkoxycarbonyl, C₁₋₄ Alkoxycarbonylalkyl, C₁₋₄ Halogenalkyl, C₆₋₁₀ Aryl, Halogen- C₆₋₁₀ Aryl, C₆₋₁₀ Aryloxy, C₆₋₁₀-Aryl-C₁₋₄ alkyl, or
R¹ represents hydrogen and
R² represents phenyl or substituted phenyl, the substituents being selected from the group consisting of Hydroxy, Amino, Halogen, Nitro, Cyano, C₁₋₄ Alkyl, C₁₋₄ Alkoxy, C₁₋₄ Alkoxyalkyl, C₁₋₄ Alkoxycarbonyl, C₁₋₄ Alkoxycarbonylalkyl, C₁₋₄ Halogenalkyl, C₆₋₁₀ Aryl, Halogen- C₆₋₁₀ Aryl, C₆₋₁₀ Aryloxy, C₆₋₁₀-Aryl-C₁₋₄ alkyl, or
R¹ represents hydrogen and .
R² represents C(O)R²¹ wherein
R²¹ represents unsubstituted or substituted C₁₋₄ alkyl, the substituents being selected from the group consisting of halogen, nitro, amino, hydroxy, C₆₋₁₀ Aryl, Halogen-C₆₋₁₀ Aryl, C₁₋₄Alkyl -C₆₋₁₀ Aryl, C₁₋₄Alkoxy -C₆₋₁₀ Aryl, C₁₋₄Halogenalkyl -C₆₋₁₀ Aryl; or
R²¹ represents unsubstituted or substituted C₁₋₄ alkoxy, the substituents being selected from the group consisting of halogen, nitro, amino, hydroxy, C₆₋₁₀ Aryl, Halogen-C₆₋₁₀ Aryl, C₁₋₄Alkyl -C₆₋₁₀ Aryl, C₁₋₄Alkoxy -C₆₋₁₀ Aryl, C₁₋₄Halogenalkyl -C₆₋₁₀ Aryl; or
R²¹ represents unsubstituted or substituted heterocycle having 3-11 ring atoms and 1-4 hetero atoms, the hetero atoms being selected from the group consisting of N, O, S, the substituents being selected from the group consisting of Oxo (=O), Hydroxy, Halogen, Amino, Nitro, Cyano, Cyano, C₁₋₄ Alkyl, C₁₋₄ Alkoxy, C₁₋₄ Alkoxyalkyl, C₁₋₄ Alkoxycarbonyl, C₁₋₄ Alkoxycarbonylalkyl, C₁₋₄ Halogenalkyl, C₆₋₁₀ Aryl, Halogen- C₆₋₁₀ Aryl, C₆₋₁₀) Aryloxy, C₆₋₁₀-Aryl-C₁₋₄ alkyl; or
R²¹ represents unsubstituted or substituted phenyl, the substituents being selected from the group consisting of Hydroxy, Amino, Halogen, Nitro, Cyano, C₁₋₄ Alkyl, C₁₋₄ Alkoxy, C₁₋₄ Alkoxyalkyl, C₁₋₄ Alkoxycarbonyl, C₁₋₄ Alkoxycarbonylalkyl, C₁₋₄ Halogenalkyl, C₆₋₁₀ Aryl, Halogen- C₆₋₁₀ Aryl, C₆₋₁₀ Aryloxy, C₆₋₁₀-Aryl-C₁₋₄ alkyl; or
R¹ and R² together with the nitrogen atom form unsubstituted or substituted heterocycle having 3-11 ring atoms and 0-3 additional hetero atoms; the hetero atoms being selected from the group consisting of N, O, S; the substituents being selected from the group consisting of Oxo (=O), Hydroxy, Halogen, Amino, Nitro, Cyano, C₁₋₄ Alkyl, C₁₋₄ Alkoxy, C₁₋₄ Alkoxyalkyl, C₁₋₄ Alkoxycarbonyl, C₁₋₄ Alkoxycarbonylalkyl, C₁₋₄ Halogenalkyl, C₆₋₁₀ Aryl, Halogen- C₆₋₁₀ Aryl, C₆₋₁₀ Aryloxy, C₆₋₁₀ -Aryl-C₁₋₄ alkyl;
R³ represents halogen or (C₁₋₄)alkyl;
n represents 1;
R⁴ represents OH and
R⁵ and R⁶ represent H
in free base or acid addition salt form.

2. A compound of formula (I) according to claim 1, wherein the compound is selected from the group consisting of
*trans*-[4-(3-Chloro-phenylethynyl)-4-hydroxy-cyclohexyl]-carbamic acid methyl ester;
*cis*-[4-(3-Chloro-phenylethynyl)-4-hydroxy-cyclohexyl]-carbamic acid methyl ester;
*trans*-[4-(4-Chloro-phenylethynyl)-4-hydroxy-cyclohexyl]-carbamic acid methyl ester;
*cis*-[4-(4-Chloro-phenylethynyl)-4-hydroxy-cyclohexyl]-carbamic acid methyl ester;
*cis*-Furan-3-carboxylic acid [4-(3-chloro-phenylethynyl)-4-hydroxy-cyclohexyl]-amide;
*trans*-Furan-3-carboxylic acid [4-(3-chloro-phenylethynyl)-4-hydroxy-cyclohexyl]-amide;
*cis*-Benzofuran-2-carboxylic acid [4-(3-chloro-phenylethynyl)-4-hydroxy-cyclohexyl]-amide;
*cis*-Furan-2-carboxylic acid [4-(3-chloro-phenylethynyl)-4-hydroxy-cyclohexyl]-amide;
*cis*-Pyridine-2-carboxylic acid [4-(3-chloro-phenylethynyl)-4-hydroxy-cyclohexyl]-amide;
*cis*-N-[4-(3-Chloro-phenylethynyl)-4-hydroxy-cyclohexyl]-nicotinamide;
*cis*- N-[4-(3-Chloro-phenylethynyl)-4-hydroxy-cyclohexyl]-isonicotinamide;
1-(3-Chloro-phenylethynyl)-4-(5-methyl-1H-pyrazol-3-ylamino)-cyclohexanol;
3-[4-(3-Chloro-phenylethynyl)-4-hydroxy-cyclohexylamino]-dihydro-furan-2-one;
4-(3-Chloro-phenylamino)-1-(3-chloro-phenylethynyl)-cyclohexanol;
1-(3-Chloro-phenylethynyl)-4-(3-methoxy-phenylamino)-cyclohexanol;
1-(3-Chloro-phenylethynyl)-4-(1H-pyrazol-3-ylamino)-cyclohexanol;
4-(4-Chloro-phenylamino)-1-(3-chlor o-phenylethynyl)-cyclohexanol;
4-(3,5-Dichloro-phenylamino)-1-(3-chlor o-phenylethynyl)-cyclohexanol;
1-(3-Chloro-phenylethynyl)-4-morpholin-4-yl-cyclohexanol;
1-(3-Chloro-phenylethynyl)-4-(1-methyl-piperidin-4-ylamino)-cyclohexanol;
4-(1-Aza-bicyclo[2.2.2]oct-3-ylamino)-1-(3-chloro-phenylethynyl)-cyclohexanol;
1-(3-Chloro-phenylethynyl)-4-(tetrahydro-pyran-4-ylamino)-cyclohexanol; and
*trans*-1-(3-Chloro-phenyfethynyl)-4-imidazol-1-yl-cyclohexanol.

3. A compound of claim 1 in free base or pharmaceutically acceptable acid addition salt form, for use as a pharmaceutical.

4. A compound of claim 1 in free base or pharmaceutically acceptable acid addition salt form, for use in the prevention, treatment or delay of progression of disorders associated with irregularities of the glutamatergic signal transmission, and of nervous system disorders mediated full or in part by mGluR5.

5. A pharmaceutical composition comprising a compound of claim 1 in free base or pharmaceutically acceptable acid addition salt form, in association with a pharmaceutical carrier or diluent.

6. The use of a compound of claim 1 in free base or pharmaceutically acceptable acid addition salt form, for the manufacture of a pharmaceutical composition designed for the prevention, treatment or delay of progression of disorders associated with irregularities of the glutamatergic signal transmission, of the gastro-intestinal and urinary tract and of nervous system disorders mediated full or in part by mGluR5.

## Patentansprüche

1. Verbindung der Formel (I) 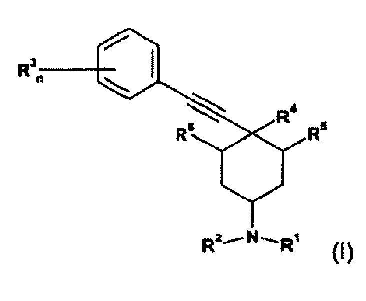 worin
R¹ für Wasserstoff und
R² für einen unsubstituierten oder substituierten Heterocyclus mit 3-11 Ringatomen und 1-4 Heteroatomen steht; wobei die Heteroatome aus der Gruppe bestehend aus N, O und S ausgewählt sind und die Substituenten aus der Gruppe bestehend aus Oxo (=O), Hydroxy, Halogen, Amino, Nitro, Cyano, C₁₋₄-Alkyl, C₁₋₄-Alkoxy, C₁₋₄-Alkoxyalkyl, C₁₋₄-Alkoxycarbonyl, C₁₋₄-Alkoxycarbonylalkyl, C₁₋₄-Halogenalkyl, C₆₋₁₀-Aryl, Halogen-C₆₋₁₀-aryl, C₆₋₁₀-Aryloxy und C₆₋₁₀-Aryl-C₁₋₄-alkyl ausgewählt sind, oder
R¹ für Wasserstoff steht und
R² für Phenyl oder substituiertes Phenyl steht, wobei die Substituenten aus der Gruppe bestehend aus Hydroxy, Amino, Halogen, Nitro, Cyano, C₁₋₄-Alkyl, C₁₋₄-Alkoxy, C₁₋₄-Alkoxyalkyl, C₁₋₄-Alkoxycarbonyl, C₁₋₄-Alkoxycarbonylalkyl, C₁₋₄-Halogenalkyl, C₆₋₁₀-Aryl, Halogen-C₆₋₁₀-aryl, C₆₋₁₀-Aryloxy und C₆₋₁₀-Aryl-C₁₋₄-alkyl ausgewählt sind, oder
R¹ für Wasserstoff steht und
R² für C (O) R²¹ steht, worin
R²¹ für unsubstituiertes oder substituiertes C₁₋₄-Alkyl steht, wobei die Substituenten aus der Gruppe bestehend aus Halogen, Nitro, Amino, Hydroxy, C₆₋₁₀-Aryl, Halogen-C₆₋₁₀-aryl, C₁₋₄-Alkyl-C₆₋₁₀ -aryl, C₁₋₄-Alkoxy-C₆₋₁₀-aryl und C₁₋₄-Halogenalkyl-C₆₋₁₀-aryl ausgewählt sind; oder
R²¹ für unsubstituiertes oder substituiertes C₁₋₄-Alkoxy steht, wobei die Substituenten aus der Gruppe bestehend aus Halogen, Nitro, Amino, Hydroxy, C₆₋₁₀-Aryl, Halogen-C₆₋₁₀-aryl, C₁₋₄-Alkyl-C₆₋₁₀-aryl, C₁₋₄-Alkoxy-C₆₋₁₀-aryl und C₁₋₄-Halogenalkyl-C₆₋₁₀-aryl ausgewählt sind; oder
R²¹ für einen unsubstituierten oder substituierten Heterocyclus mit 3-11 Ringatomen und 1-4 Heteroatomen steht; wobei die Heteroatome aus der Gruppe bestehend aus N, O und S ausgewählt sind und die Substituenten aus der Gruppe bestehend aus Oxo (=O), Hydroxy, Halogen, Amino, Nitro, Cyano, C₁₋₄-Alkyl, C₁₋₄-Alkoxy, C₁₋₄-Alkoxyalkyl, C₁₋₄-Alkoxycarbonyl, C₁₋₄-Alkoxycarbonylalkyl, C₁₋₄-Halogenalkyl, C₆₋₁₀-Aryl, Halogen-C₆₋₁₀-aryl, C₆₋₁₀-Aryloxy und C₆₋₁₀-Aryl-C₁₋₄-alkyl ausgewählt sind; oder
R²¹ für unsubstituiertes oder substituiertes Phenyl steht, wobei die Substituenten aus der Gruppe bestehend aus Hydroxy, Amino, Halogen, Nitro, Cyano, C₁₋₄-Alkyl, C₁₋₄-Alkoxy, C₁₋₄-Alkoxyalkyl, C₁₋₄-Alkoxycarbonyl, C₁₋₄-Alkoxycarbonylalkyl, C₁₋₄-Halogenalkyl, C₆₋₁₀-Aryl, Halogen-C₆₋₁₀-aryl, C₆₋₁₀-Aryloxy und C₆₋₁₀-Aryl-C₁₋₄-alkyl ausgewählt sind; oder
R¹ und R² gemeinsam mit dem Stickstoffatom einen unsubstituierten oder substituierten Heterocyclus mit 3-11 Ringatomen und 0-3 zusätzlichen Heteroatomen bilden; wobei die Heteroatome aus der Gruppe bestehend aus N, O und S ausgewählt sind und die Substituenten aus der Gruppe bestehend aus Oxo (=0), Hydroxy, Halogen, Amino, Nitro, Cyano, C₁₋₄-Alkyl, C₁₋₄-Alkoxy, C₁₋₄-Alkoxyalkyl, C₁₋₄-Alkoxycarbonyl, C₁₋₄-Alkoxycarbonylalkyl, C₁₋₄-Halogenalkyl, C₆₋₁₀-Aryl, Halogen-C₆₋₁₀-aryl, C₆₋₁₀-Aryloxy und C₆₋₁₀-Aryl-C₁₋₄-alkyl ausgewählt sind;
R³ für Halogen oder C₁₋₄-Alkyl steht;
n für 1 steht;
R⁴ für OH steht und
R⁵ und R⁶ für H stehen;
in Form der freien Base oder eines Säureadditionssalzes.

2. Verbindung der Formel (I) nach Anpruch 1, ausgewählt aus der Gruppe bestehend aus
*trans*-[4-(3-Chlorphenylethinyl)-4-hydroxycyclohexyl]carbamidsäuremethylester;
*cis*-[4-(3-Chlorphenylethinyl)-4-hydroxycyclohexyl]carbamidsäuremethylester;
*trans*-[4-(4-Chlorphenylethinyl)-4-hydroxycyclohexyl]carbamidsäuremethylester;
*cis*-[4-(4-Chlorphenylethinyl)-4-hydroxycyclohexyl]carbamidsäuremethylester;
*cis*-Furan-3-carbonsäure[4-(3-chlorphenylethinyl)-4-hydroxycyclohexyl]amid;
*trans*-Furan-3-carbonsäure[4-(3-chlorphenylethinyl)-4-hydroxycyclohexyl]amid;
*cis*-Benzofuran-2-carbonsäure[4-(3-chlorphenylethinyl)-4-hydroxycyclohexyl]amid;
*cis*-Furan-2-carbonsäure[4-(3-chlorphenylethinyl)-4-hydroxycyclohexyl]amid;
*cis*-Pyridin-2-carbonsäure[4-(3-chlorphenylethinyl)-4-hydroxycyclohexyl]amid;
*cis*-N-[4-(3-Chlorphenylethinyl)-4-hydroxycyclohexyl]nicotinamid;
*cis*-N-[4-(3-Chlorphenylethinyl)-4-hydroxycyclohexyl]isonicotinamid;
1-(3-Chlorphenylethinyl)-4-(5-methyl-1H-pyrazol-3-ylamino)cyclohexanol;
3-[4-(3-Chlorphenylethinyl)-4-hydroxycyclohexylamino]dihydrofuran-2-on;
4-(3-Chlorphenylamino)-1-(3-chlor-o-phenylethinyl)cyclohexanol;
1-(3-Chlorphenylethinyl)-4-(3-methoxyphenylamino)-cyclohexanol;
1-(3-Chlorphenylethinyl)-4-(1H-pyrazol-3-ylamino)-cyclohexanol;
4-(4-Chlorphenylamino)-1-(3-chlor-o-phenylethinyl)cyclohexanol;
4-(3,5-Dichlorphenylamino)-1-(3-chlor-o-phenylethinyl)cyclohexanol;
1-(3-Chlorphenylethinyl)-4-morpholin-4-ylcyclohexanol;
1-(3-Chlorphenylethinyl)-4(1-methylpiperidin-4-ylamino)cyclohexanol;
4-(1-Azabicyclo[2.2.2]oct-3-ylamino)-1-(3-chlorphenylethinyl)cyclohexanol;
1-(3-Chlorphenylethinyl)-4-(tetrahydropyran-4-yl-amino)cyclohexanol und
*trans*-1-(3-Chlorphenylethinyl)-4-imidazol-1-yl-cyclohexanol.

3. Verbindung nach Anspruch 1 in Form der freien Base oder eines pharmazeutisch annehmbaren Säureadditionssalzes zur Verwendung als Pharmazeutikum.

4. Verbindung nach Anspruch 1 in Form der freien Base oder eines pharmazeutisch annehmbaren Säureadditionssalzes zur Verwendung bei der Prävention, Behandlung oder Verzögerung des Fortschreitens von Störungen, die mit Unregelmäßigkeiten der glutamatergen Signalübertragung assoziiert sind, und von Störungen des Nervensystems, die ganz oder teilweise durch mGluR5 vermittelt werden.

5. Pharmazeutische Zusammensetzung, die eine Verbindung nach Anspruch 1 in Form der freien Base oder eines pharmazeutisch annehmbaren Säureadditionssalzes zusammen mit einem pharmazeutischen Träger oder Verdünnungsmittel enthält.

6. Verwendung einer Verbindung nach Anspruch 1 in Form der freien Base oder eines pharmazeutisch annehmbaren Säureadditionssalzes zur Herstellung einer pharmazeutischen Zusammensetzung zur Prävention, Behandlung oder Verzögerung des Fortschreitens von Störungen, die mit Unregelmäßigkeiten der glutamatergen Signalübertragung assoziiert sind, des Magen-Darm- und Harntrakts und von Störungen des Nervensystems, die ganz oder teilweise durch mGluR5 vermittelt werden.

## Revendications

1. Composé de formule (I) 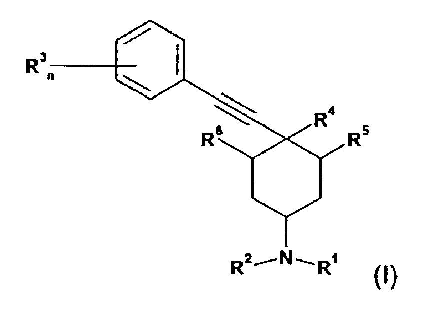 dans laquelle
R¹ représente hydrogène et
R² représente un hétérocycle non substitué ou substitué ayant 3 à 11 atomes du cycle et 1 à 4 hétéroatomes ; les hétéroatomes étant choisis dans le groupe constitué de N, O, S, les substituants étant choisis dans le groupe constitué d¹ Oxo (=O), Hydroxy, Halogène, Amino, Nitro, Cyano, C₁₋₄ Alkyle, C₁₋₄ Alcoxy, C₁₋₄ Alcoxyalkyle, C₁₋₄ Alcoxycarbonyle, C₁₋₄ Alcoxycarbonylalkyle, C₁-₄ Halogénoalkyle, C₆₋₁₀ Aryle, Halogène- C₆-₁₀ Aryle, C₆₋₁₀ Aryloxy, C₆₋₁₀-Aryl-C₁₋₄ alkyle, ou
R¹ représente hydrogène et
R² représente phényle ou phényle substitué, les substituants étant choisis dans le groupe constitué d'Hydroxy, Amino, Halogène, Nitro, Cyano, C₁-₄ Alkyle, C₁₋₄ Alcoxy, C₁₋₄ Alcoxyalkyle, C₁₋₄ Alcoxycarbonyle, C₁₋₄ Alcoxycarbonylalkyle, C₁₋₄ Halogénoalkyle, C₆₋₁₀ Aryle, Halogène- C₆-₁₀ Aryle, C₆₋₁₀ Aryloxy, C₆₋₁₀-Aryl-C₁₋₄ alkyle, ou
R¹ représente hydrogène et
R² représente C(O)R²¹ où
R²¹ représente C₁₋₄ alkyle non substitué ou substitué, les substituants étant choisis dans le groupe constitué d'halogène, nitro, amino, hydroxy, C₆₋₁₀ Aryle, Halogène-C₆₋₁₀ Aryle, C₁₋₄ Alkyl-C₆₋₁₀ Aryle, C₁₋₄ Alcoxy-C₆₋₁₀ Aryle, C₁₋₄ Halogénoalkyl-C₆₋₁₀ Aryle ; ou
R²¹ représente C₁₋₄ alcoxy non substitué ou substitué, les substituants étant choisis dans le groupe constitué d'halogène, nitro, amino, hydroxy, C₆₋₁₀ Aryle, Halogène-C₆₋₁₀ Aryle, C₁₋₄ Alkyl-C₆₋₁₀ Aryle, C₁₋₄ Alcoxy-C₆₋₁₀ Aryle, C₁₋₄ Halogénoalkyl-C₆-₁₀ Aryle ; ou
R²¹ représente un hétérocycle non substitué ou substitué ayant 3 à 11 atomes du cycle et 1 à 4 hétéroatomes, les hétéroatomes étant choisis dans le groupe constitué de N, O, S, les substituants étant choisis dans le groupe constitué d'Oxo (=O), Hydroxy, Halogène, Amino, Nitro, Cyano, C₁₋₄ Alkyle, C₁₋₄ Alcoxy, C₁₋₄ Alcoxyalkyle, C₁₋₄ Alcoxycarbonyle, C₁₋₄ Alcoxycarbonylalkyle, C₁₋₄ Halogénoalkyle, C₆₋₁₀ Aryle, Halogène- C₆₋₁₀ Aryle, C₆₋₁₀ Aryloxy, C₆₋₁₀-Aryl-C₁₋₄ alkyle ; ou
R²¹ représente phényle non substitué ou substitué, les substituants étant choisis dans le groupe constitué d'Hydroxy, Amino, Halogène, Nitro, Cyano, C₁₋₄ Alkyle, C₁₋₄ Alcoxy, C₁₋₄ Alcoxyalkyle, C₁₋₄ Alcoxycarbonyle, C₁₋₄ Alcoxycarbonylalkyle, C₁₋₄ Halogénoalkyle, C₆₋₁₀ Aryle, Halogène- C₆₋₁₀ Aryle, C₆₋₁₀ Aryloxy, C₆₋₁₀-Aryl-C₁₋₄ alkyle ; ou
R¹ et R² ensemble avec l'atome d'azote forment un hétérocycle non substitué ou substitué ayant 3 à 11 atomes du cycle et 0 à 3 hétéroatomes supplémentaires ; les hétéroatomes étant choisis dans le groupe constitué de N, O, S ; les substituants étant choisis dans le groupe constitué d'Oxo (=O), Hydroxy, Halogène, Amino, Nitro, Cyano, C₁₋₄ Alkyle, C₁₋₄ Alcoxy, C₁₋₄ Alcoxyalkyle, C₁₋₄ Alcoxycarbonyle, C₁₋₄ Alcoxycarbonylalkyle, C₁₋₄ Halogénoalkyle, C₆₋₁₀ Aryle, Halogène- C₆₋₁₀ Aryle, C₆₋₁₀ Aryloxy, C₆₋₁₀-Aryl-C₁₋₄ alkyle ;
R³ représente (C₁₋₄)alkyle ou halogène,
n représente 1 ;
R⁴ représente OH et
R⁵ et R⁶ représentent H
sous forme de base libre ou de sel d'addition d'acide.

2. Composé de formule (I) selon la revendication 1, **caractérisé en ce que** le composé est choisi dans le groupe constitué de
ester méthylique d'acide trans-[4-(3-chlorophényléthynyl)-4-hydroxy-cyclohexyl]-carbamique ;
ester méthylique d'acide cis-[4-(3-chlorophényléthynyl)-4-hydroxy-cyclohexyl]-carbamique ;
ester méthylique d'acide trans-[4-(4-chlorophényléthynyl)-4-hydroxy-cyclohexyl]-carbamique ;
ester méthylique d'acide cis-[4-(4-chlorophényléthynyl)-4-hydroxy-cyclohexyl]-carbamique ;
[4-(3-chloro-phényléthynyl)-4-hydroxy-cyclohexyl]-amide d'acide cis-furane-3-carboxylique ;
[4-(3-chloro-phényléthynyl)-4-hydroxy-cyclohexyl]-amide d'acide trans-furane-3-carboxylique ;
[4-(3-chloro-phényléthynyl)-4-hydroxy-cyclohexyl]-amide d'acide cis-benzofurane-2-carboxylique ;
[4-(3-chloro-phényléthynyl)-4-hydroxy-cyclohexyl]-amide d'acide cis-furane-2-carboxylique ;
[4-(3-chloro-phényléthynyl)-4-hydroxy-cyclohexyl]-amide d'acide *cis*-pyridine-2-carboxylique ;
cis-N-[4-(3-chloro-phényléthynyl)-4-hydroxycyclohexyl]-nicotinamide ;
cis-N-[4-(3-chloro-phényléthynyl)-4-hydroxycyclohexyl]-isonicotinamide ;
1-(3-chloro-phényléthynyl)-4-(5-méthyl-1H-pyrazol-3-ylamino)-cyclohexanol ;
3-[4-(3-chloro-phényléthynyl)-4-hydroxycyclohexylamino]-dihydro-furan-2-one ;
4-(3-chloro-phénylamino)-1-(3-chlorophényléthynyl)-cyclohexanol ;
1-(3-chloro-phényléthynyl)-4-(3-méthoxyphénylamino)-cyclohexanol ;
1-(3-chloro-phényléthynyl)-4-(1H-pyrazol-3-ylamino)-cyclohexanol ;
4-(4-chloro-phénylamino)-1-(3-chlorophényléthynyl)-cyclohexanol ;
4-(3,5-dichloro-phénylamino)-1-(3-chlorophényléthynyl)-cyclohexanol ;
1-(3-chloro-phényléthynyl)-4-morpholin-4-yl-cyclohexanol ;
1-(3-chloro-phényléthynyl)-4-(1-méthyl-pipéridin-4-ylamino)-cyclohexanol ;
4-(1-azabicyclo[2,2,2]oct-3-ylamino)-1-(3-chlorophényléthynyl)-cyclohexanol ;
1-(3-chloro-phényléthynyl)-4-(tétrahydro-pyran-4-ylamino)-cyclohexanol ; et
*trans*-1-(3-chloro-phényléthynyl)-4-imidazol-1-yl-cyclohexanol.

3. Composé selon la revendication 1, sous forme de base libre ou de sel d'addition d'acide pharmaceutiquement acceptable, destiné à être utilisé comme produit pharmaceutique.

4. Composé selon la revendication 1, sous forme de base libre ou de sel d'addition d'acide pharmaceutiquement acceptable, destiné à être utilisé pour prévenir, traiter ou retarder la progression de troubles associés à des irrégularités de la transmission du signal glutamatergique, et de troubles du système nerveux médiés totalement ou en partie par le mGluR5.

5. Composition pharmaceutique comprenant un composé selon la revendication 1, sous forme de base libre ou de sel d'addition d'acide pharmaceutiquement acceptable, en association avec un support ou un diluant pharmaceutique.

6. Utilisation d'un composé selon la revendication 1, sous forme de base libre ou de sel d'addition d'acide pharmaceutiquement acceptable, pour la fabrication d'une composition pharmaceutique conçue pour prévenir, traiter ou retarder la progression de troubles associés à des irrégularités de la transmission du signal glutamatergique, de troubles de la voie gastro-intestinale et urinaire et de troubles du système nerveux médiés totalement ou en partie par le mGluR5.
